# DEMANDE DE BREVET EUROPEEN

(11) **EP 4 116 429 A1**
(43) Date de publication de la demande: **11.01.2023**
(21) Numéro de dépôt: 22182863.5
(22) Date de dépôt: 04.07.2022
(51) Int. Cl.: C12Q 1/02, G01N 1/28

(54) **PROCÉDÉ D'ANALYSE D'UN ÉCHANTILLON POUR Y DÉTECTER LA PRÉSENCE D'UNE FORME ACTIVE D'UNE ESPÈCE BIOLOGIQUE**

(30) Priorité: 07.07.2021 FR 2107363
(71) Demandeur: Commissariat à l'énergie atomique et aux énergies alternatives, 75015 Paris (FR)
(72) Inventeur: BOURDAT, Anne-Gaëlle, 38054 GRENOBLE (FR); DESVERGNE, Caroline, 38054 GRENOBLE (FR)
(74) Mandataire: INNOV-GROUP

(57) **Abrégé**

L'invention concerne un procédé d'analyse d'un échantillon (ECH) afin de détecter la présence d'une espèce biologique sous une première forme, dite forme active (F2), dans ledit échantillon, ledit échantillon comportant ladite espèce biologique sous une deuxième forme, dite forme dormante (F1) de ladite espèce biologique, distincte de la forme active (F2). Le procédé consiste à séparer ledit échantillon (ECH) en deux parties (A, B) distinctes de volumes identiques, dites première partie (A) de l'échantillon et deuxième partie (B) de l'échantillon, à déterminer une première quantité (Q1) d'espèces biologiques dans la première partie (A) de l'échantillon et une deuxième quantité (Q2) d'espèces biologiques dans la deuxième partie de l'échantillon et à comparer la première quantité (Q1) obtenue et la deuxième quantité (Q2) obtenue, en vue de déduire la présence ou l'absence de la forme active (F2) de l'espèce biologique dans l'échantillon.

## Description

### Domaine technique de l'invention

La présente invention se rapporte à un procédé d'analyse d'un échantillon pour y détecter la présence d'une forme active d'une espèce biologique.

### Etat de la technique

Dans le domaine de l'agriculture, certaines espèces biologiques peuvent exister à la fois sous une forme dite dormante et inoffensive, c'est-à-dire résistante à toute action extérieure et non active, non virulente, ainsi que sous une forme dite active, susceptible d'entraîner des désagréments dans les cultures. Cette situation peut également se présenter dans le domaine de la santé d'un être vivant. Les deux formes de l'espèce biologique possèdent le même génome et le même type de protéines génériques mais elles n'ont pas le même effet sur les cultures ou sur la santé de l'être vivant.

Un cas particulier connu est celui du mildiou qui est une maladie courante présente en agriculture et particulièrement destructrice. Le pathogène responsable de cette maladie peut être à la fois dormant et actif. Il est par exemple susceptible de se développer sur les feuilles des végétaux et sur les grappes de raisins. Il est actuellement traité par des applications répétées et systématiques de fongicide, qui entraînent pollution environnementale, toxicité et développement de souches plus résistantes.

Pour adapter les traitements et les appliquer que lorsque cela est nécessaire, il faut pouvoir savoir si le pathogène est présent sous sa forme active ou sous sa forme dormante.

Pour cela, il existe aujourd'hui différentes solutions, plus ou moins complexes et plus ou moins efficaces :
- Installation de stations météo dans les champs et application de modèles prédictifs destinés à calculer les risques d'apparition du pathogène dans sa forme active ;
- Analyse de prélèvements réalisés sur le site et détection biomoléculaire;
- Détection optique par fluorescence type "Agrolase" (Marque déposée) ;

Dans la première solution proposée, la mise en place de modèles prédictifs est longue et leur exécution peut s'avérer peu fiable.

Dans la deuxième solution, la détection biomoléculaire permet de détecter la présence de l'espèce biologique et éventuellement la quantité d'espèces biologiques présentes, mais pas de savoir si elle est présente sous sa forme dormante ou sous sa forme active.

Dans la troisième solution, le système est particulièrement lourd à mettre en place et suppose un suivi en continu.

Il existe donc un besoin de disposer d'une solution qui permette de détecter la présence d'une forme active d'une espèce biologique, qui soit fiable et facile à mettre en œuvre et à déployer, sans utiliser de matériel lourd et encombrant.

Le procédé de l'invention permet de répondre à ce besoin.

### Exposé de l'invention

L'invention consiste ainsi en un procédé d'analyse d'un échantillon afin de détecter la présence d'une espèce biologique sous une première forme, dite forme active, dans ledit échantillon, ledit échantillon comportant ladite espèce biologique sous une deuxième forme, dite forme dormante de ladite espèce biologique, distincte de la forme active, le procédé, à partir d'un même échantillon, comporte des étapes de :
- Séparation dudit échantillon en deux parties distinctes de volumes identiques, dites première partie de l'échantillon et deuxième partie de l'échantillon,
- Première concentration des espèces biologiques contenues dans la première partie de l'échantillon,
- Traitement doux en flux ou aucun traitement de la première partie de l'échantillon,
- Première détection quantitative de ladite espèce biologique dans la première partie de l'échantillon en vue de déterminer une première quantité d'espèces biologiques dans la première partie de l'échantillon,
- Deuxième concentration des espèces biologiques contenues dans la deuxième partie de l'échantillon,
- Traitement actif en flux de la deuxième partie de l'échantillon,
- Deuxième détection quantitative de ladite espèce biologique dans la deuxième partie de l'échantillon en vue de déterminer une deuxième quantité d'espèces biologiques dans la deuxième partie de l'échantillon,
- Comparaison entre la première quantité obtenue et la deuxième quantité obtenue, en vue de déduire la présence ou l'absence de la forme active de l'espèce biologique dans l'échantillon.

Selon une particularité, le traitement actif de la deuxième partie de l'échantillon comporte au moins un traitement chimique ou biochimique, réalisé par injection en flux d'une substance destructrice de la forme active de l'espèce biologique à la deuxième partie de l'échantillon après concentration, en vue de casser sa membrane externe et libérer ses acides nucléiques.

Selon une autre particularité, le traitement actif comporte également un ou plusieurs des traitements suivants, ou une combinaison d'au moins deux de ces traitements :
- Traitement thermique,
- Traitement mécanique,
- Traitement optique.

Selon une autre particularité, le traitement actif est réalisé par lyse des espèces biologiques contre une surface rugueuse.

Selon une autre particularité, le traitement thermique consiste en un chauffage par des moyens de chauffage de la deuxième partie de l'échantillon à une température donnée et pendant une durée déterminée.

Selon une autre particularité, le traitement optique consiste à exposer la deuxième partie de l'échantillon à un signal lumineux émis à une longueur d'onde donnée et pendant une durée déterminée.

Selon une autre particularité, la première concentration et la deuxième concentration sont mises en œuvre en employant un composant micro-fluidique qui comporte :
- Un boîtier comportant au moins une ouverture,
- Un premier canal ménagé dans ledit boîtier,
- Un deuxième canal ménagé dans ledit boîtier,
- Une chambre dans laquelle débouche le premier canal et le deuxième canal,
- Un filtre séparant ladite chambre en deux espaces distincts de manière à définir un premier espace dans lequel débouche ledit premier canal d'injection et un deuxième espace dans lequel débouche ledit deuxième canal, ledit filtre ayant une porosité adaptée pour retenir les espèces biologiques présentes dans l'échantillon.

Selon une autre particularité, la première concentration et la deuxième concentration sont mises en œuvre de manière simultanée dans deux composants micro-fluidiques identiques en parallèle.

Selon une autre particularité, le traitement doux et/ou ledit traitement actif sont mis en œuvre dans ledit composant micro-fluidique, par injection en flux à travers le premier canal du composant.

Selon une autre particularité, la première détection quantitative et la deuxième détection quantitative sont réalisées par amplification biomoléculaire.

Selon une autre particularité, l'échantillon est obtenu après une étape de broyage et d'extraction d'un prélèvement à analyser.

Il faut noter que le procédé de l'invention est adapté pour détecter la présence de la forme active d'espèces biologiques, telles que :
- Bactéries sous une forme végétative (active) ou sporulée (dormante) ;
- Pathogènes fongiques à cycle de sporulation ;

Il est ainsi possible de détecter l'apparition de certaines maladies telles que Mildiou, Pseudoperonospora, oïdium, botrytis...

### Brève description des figures

D'autres caractéristiques et avantages vont apparaître dans la description détaillée qui suit faite en regard des dessins annexés dans lesquels :
- La figure 1 représente un diagramme illustrant les étapes mises en œuvre dans le procédé de l'invention, selon une première variante de réalisation ;
- La figure 2 représente un diagramme illustrant les étapes mises en œuvre dans le procédé de l'invention, selon une deuxième variante de réalisation ;
- La figure 3 représente de manière schématique le composant micro-fluidique pouvant être employé pour mettre en œuvre le procédé de l'invention, dans la première variante et la deuxième variante de réalisation ;

### Description détaillée d'au moins un mode de réalisation

Pour la suite de la description, une espèce biologique ciblée peut être présente dans l'échantillon sous une forme dite dormante (ci-après F1) et sous une forme dite active (ci-après F2). La forme dormante F1 est la forme inoffensive et résistante de l'espèce biologique alors que sa forme active F2 est une forme active et destructrice. Les termes "inoffensive" et "destructrice" doivent bien entendu être compris en rapport avec le milieu dans lequel l'espèce biologique est présente, ce milieu pouvant être un être vivant, un végétal, une matrice alimentaire...

Le principe de détection utilisé dans l'invention s'appuie en outre sur le fait que l'espèce biologique est souvent plus fragile dans sa forme active F2 que dans sa forme dormante F1, dans le sens où, lorsqu'un traitement actif est mené sur l'échantillon, les molécules d'ADN de la forme active F2 ont tendance à se casser plus facilement que celles de la forme dormante F1.

L'invention vise ainsi à proposer un procédé permettant de détecter la présence d'une forme active F2 d'une espèce biologique dans un échantillon ECH.

Pour cela, le procédé consiste principalement à :
- Diviser un échantillon ECH de départ en deux parties A, B de quantité identique,
- Réaliser un traitement doux T1 ou aucun traitement sur une première partie A de l'échantillon et un traitement dit actif T2 sur une deuxième partie B de l'échantillon,
- Concentrer les espèces biologiques présentes dans chaque partie de l'échantillon,
- Détecter quantitativement l'espèce biologique cible dans chacune des deux parties de l'échantillon,
- Effectuer une comparaison des résultats obtenus pour chaque partie de l'échantillon en effectuant un traitement différentiel.

Selon le ratio obtenu entre la quantité Q1 d'espèces biologiques présentes dans la première partie A de l'échantillon et la quantité Q2 d'espèces biologiques présentes dans la deuxième partie B de l'échantillon, on peut alors déterminer si l'espèce biologique est présente et si elle l'est dans sa forme active F2 et dormante F1, ou uniquement dans sa forme dormante F1.

Le procédé de l'invention est notamment adapté pour détecter la présence de la forme active F2 d'espèces biologiques, telles que :
- Bactéries sous une forme végétative (active) ou sporulée (dormante) ;
- Pathogènes fongiques à cycle de sporulation ;

Il est ainsi possible de détecter l'apparition de certaines maladies telles que Mildiou, Pseudoperonospora, oïdium, botrytis...

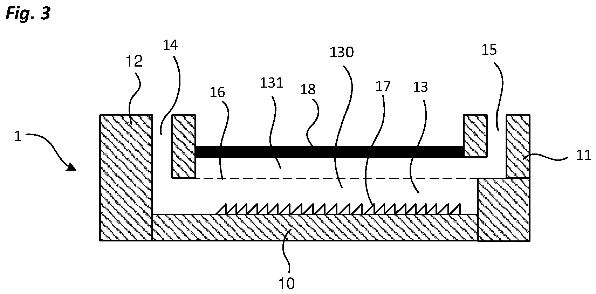

Pour mettre en œuvre le procédé de l'invention, on utilise avantageusement un composant micro-fluidique représenté sur la figure 3.

Ce composant micro-fluidique 1 comporte un boîtier comprenant une paroi inférieure 10, une paroi latérale 11 et une paroi supérieure 12. Toutes les parois du boîtier seront préférentiellement réalisées dans un même matériau. Ce matériau sera notamment apte à pouvoir subir un chauffage dans une fourchette de température comprise entre 20°C et 100°C. Préférentiellement, certaines parois du boîtier seront réalisées dans un matériau transparent. Préférentiellement, le matériau employé sera un plastique, par exemple de type PMMA (Poly(Méthacrylate de Méthyle)) ou COC (Cyclic Olefin Copolymer).

Le composant 1 comporte une chambre 13 ménagée dans le boîtier. Cette chambre représente l'emplacement dans lequel peuvent être effectuées à la fois la purification/concentration et la détection des espèces biologiques cibles. La chambre 13 est fermée vers le bas par la paroi inférieure du boîtier.

Le composant micro-fluidique comporte un premier canal 14 ménagé dans le boîtier et agencé pour injecter des fluides dans la chambre ou pour évacuer des fluides en dehors de la chambre. Le premier canal 14 comporte une première extrémité comportant une ouverture ménagée par exemple à travers la paroi supérieure 12 du boîtier et une deuxième extrémité qui débouche dans ladite chambre 13. La première extrémité du premier canal 14 est par exemple agencée verticalement et sa deuxième extrémité débouche par exemple horizontalement dans la chambre 13. La première extrémité du premier canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type "luer" pour y connecter une seringue ou adaptée pour y connecter un circuit fluidique.

Le composant comporte un deuxième canal 15 ménagé dans le boîtier. Ce deuxième canal 15 comporte également une première extrémité qui communique avec l'extérieur, formant une ouverture réalisée par exemple à travers la paroi supérieure du boîtier et une deuxième extrémité qui communique avec l'espace formé par la chambre 13. Par ce deuxième canal 15, on peut également injecter des fluides dans ladite chambre ou évacuer des fluides en dehors de ladite chambre. Sa première extrémité est par exemple agencée verticalement et sa deuxième extrémité horizontalement. La chambre 13 est placée entre le premier canal 14 et le deuxième canal 15. De manière identique, la première extrémité de ce deuxième canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type "luer" pour y connecter une seringue ou adaptée pour y connecter un circuit fluidique.

Vers le haut, la chambre 13 peut être fermée par une membrane 18 avantageusement souple et étirable, préférentiellement transparente. La paroi supérieure 12 du boîtier du dispositif comporte ainsi une ouverture qui est recouverte de manière hermétique par ladite membrane 18. Ladite membrane est ainsi ancrée dans le boîtier par toute solution de fixation adaptée, par exemple par collage. Cette membrane 18 sera par exemple composée d'un film, par exemple un film autocollant de type PET, d'épaisseur, de dimensions et de constitution adaptées pour se déformer de manière élastique, par rapport à ses points d'ancrage, notamment jusqu'au fond de la chambre 13.

Par le terme "transparent", on entend que le matériau employé est au moins partiellement transparent à la lumière visible, à la fluorescence ou à la luminescence, de manière à laisser passer au moins 80% de cette lumière. Il faut ainsi comprendre qu'il sera suffisamment transparent pour voir l'intérieur de la chambre 13, au moins le deuxième espace situé au-dessus du filtre 16 évoqué ci-dessous.

Le composant micro-fluidique 1 comporte un filtre 16 agencé dans ladite chambre 13 et séparant ladite chambre 13 en deux espaces. Les deux espaces sont par exemple superposés et désignés ainsi espace inférieur 130 situé sous le filtre et espace supérieur 131 situé au-dessus du filtre et sous la membrane 18. Ce filtre 16 est préférentiellement réalisé en tout ou partie sous la forme d'un film souple et fin, maintenu dans l'espace formé par la chambre de manière à ne permettre le passage d'un espace à l'autre que par les pores du filtre 16. Le film présente avantageusement une déformabilité élastique lui permettant de s'étirer lors de l'exercice d'une force d'appui dans une direction sensiblement verticale, cette déformabilité élastique ayant un niveau suffisant pour atteindre la surface inférieure de la chambre 13. Le filtre 16 présente un diamètre moyen de pores compris entre 0.2 µm et 50 µm, par exemple compris entre 0.2 µm et 1 µm pour la séparation de microorganismes. Le diamètre des pores est bien entendu adapté pour assurer une séparation entre différentes espèces biologiques présentes dans l'échantillon. Le filtre 16 sera par exemple composé d'un film d'épaisseur, de dimensions et de constitution adaptée pour se déformer jusqu'au fond de la chambre 13 par rapport à ses points d'ancrage. Selon un mode de réalisation particulier, le filtre pourra être aussi réalisé dans un matériau transparent, par exemple avec les mêmes caractéristiques de transparence que la membrane. Pour des bactéries, le filtre peut présenter un diamètre de pores allant de 0.2 à 2µm pour retenir les bactéries.

L'un des traitements actifs pouvant être appliqué à une partie de l'échantillon peut consister en une lyse des espèces biologiques présentes dans l'échantillon. En cas de lyse, le composant micro-fluidique peut avantageusement comporter une surface d'appui rugueuse 17 agencée sur le fond de la chambre 13. Cette surface d'appui rugueuse 17 s'étend sur une partie majoritaire du fond de la chambre. Elle comporte un paramètre de rugosité de surface moyen compris entre 0.01µm et 10 µm, préférentiellement compris entre 0.2 µm et 3 µm. Cette surface d'appui rugueuse 17 est destinée à permettre une lyse mécanique des espèces biologiques présentes dans l'échantillon biologique placé dans le dispositif. Préférentiellement, la lyse mécanique est réalisée en broyant lesdites espèces biologiques, par abrasion sur ladite surface d'appui rugueuse. L'opération de broyage est mise en œuvre par un mouvement de friction des espèces biologiques contre la surface d'appui rugueuse, en employant un organe de broyage adapté. Cet organe sera par exemple une spatule ou une tige, par exemple en matériau plastique ou métallique. Cet organe est appliqué depuis l'extérieur de la chambre 13 et son extrémité est appliquée contre la surface externe de la membrane 18 de manière à étirer la membrane 18 et le filtre vers le fond de la chambre et ainsi frictionner les espèces biologiques présentes dans un échantillon contre la surface d'appui rugueuse 17.

En cas de traitement actif de type thermique, le boîtier peut avantageusement intégrer des moyens de chauffage de l'espace interne de la chambre, composés par exemple d'au moins une résistance chauffante. La résistance est par exemple fixée sous la paroi inférieure du boîtier. Une source d'alimentation sera par exemple prévue pour alimenter la résistance. La source d'alimentation comportera par exemple une ou plusieurs piles électriques, fournissant suffisamment d'énergie pour chauffer la chambre à une température comprise dans la fourchette définie ci-dessus, c'est-à-dire de 20°C à 100°C. Bien entendu, d'autres moyens de chauffage pourraient être employés, comprenant par exemple une encre conductrice déposée par imprimerie ou sérigraphie sous la paroi inférieure du boîtier.

Ainsi, pour résumer, le composant micro-fluidique peut avantageusement comporter la structure "multicouches" suivante :
- Optionnellement, une surface inférieure d'appui rugueuse 17,
- Un espace inférieur 130 de la chambre 13, situé au-dessus de la surface d'appui rugueuse 17,
- Un filtre 16, avantageusement souple et étirable situé au-dessus de l'espace inférieur 130,
- Un espace supérieur 131 de la chambre 13 situé au-dessus du filtre 16,
- Une membrane 18, avantageusement souple et étirable située au-dessus de l'espace supérieur 131, fermant hermétiquement la chambre et accessible depuis l'extérieur du dispositif.

La paroi inférieure du composant et la membrane peuvent être réalisés dans des matériaux transparents, notamment pour mettre en œuvre une détection par amplification biomoléculaire.

De manière non limitative, le composant micro-fluidique peut présenter les caractéristiques dimensionnelles suivantes :
- Un premier canal 14 composé d'un canal d'entrée de 1mm de diamètre x 3mm de hauteur, puis d'un canal de section rectangulaire de 1mm×150µm de 3mm de long ;
- Une chambre 13 composée d'un espace inférieur 130 pour concentration/lyse qui présente un diamètre de 8mm×150µm de hauteur et d'un espace supérieur pour élution d'un diamètre 8mm×300µm de hauteur ;
- Filtre 16 de porosité adaptée à la cible à retenir (virus, levure, moisissure...), par exemple avec une porosité de 0.2 à 2 µm pour retenir les bactéries ou de 10 à 100 nm pour retenir les virus ;
- Un deuxième canal 15 composé d'un canal de section rectangulaire de 1mm × 150µm et de 3mm de long, puis d'un canal de 1mm de diamètre x3mm de hauteur ;

Ce composant permet notamment d'injecter l'échantillon en flux et de le traiter en flux. Par l'expression "en flux" on entend que l'échantillon est injecté sous forme liquide dans le composant de manière continue, via son premier canal 14, sans sortir l'échantillon du composant. Il en est de même des solutions de traitement qui lui sont appliquées, celles-ci étant injectées sous forme fluidique en continu dans le composant, via son premier canal 14, sans avoir à retirer l'échantillon présent dans le composant. Grâce au filtre 16, les espèces biologiques cibles sont maintenues dans l'espace inférieur 130 de la chambre 13 du composant.

Le terme "flux" s'oppose au terme "reflux", qui consiste pour sa part à injecter un fluide via le deuxième canal 15 du composant, pour chasser l'échantillon en dehors du composant, via le premier canal 14.

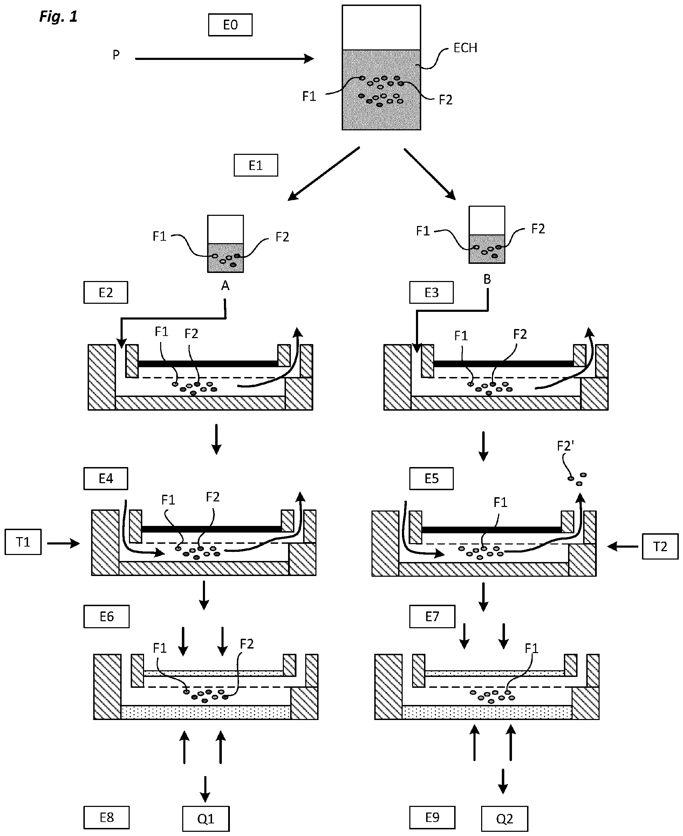

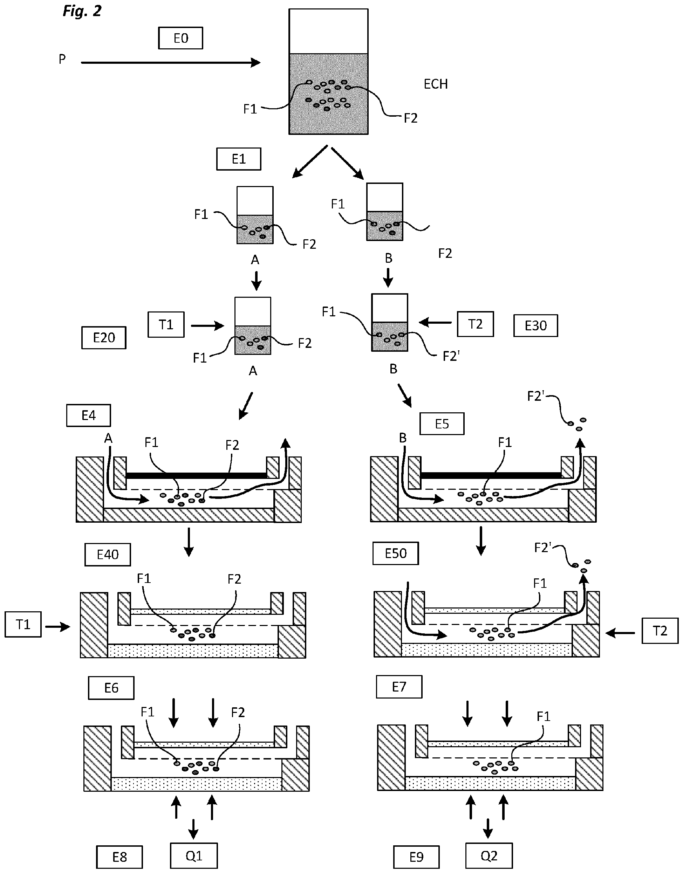

En utilisant ce composant micro-fluidique, le procédé peut être mis en œuvre selon les deux variantes représentées sur les figures 1 et 2.

Il faut noter que le composant micro-fluidique présente notamment l'avantage d'optimiser la quantité d'espèces biologiques cibles obtenues et de les concentrer au maximum dans l'espace inférieur 130 de la chambre 13 du composant 1.

Pour les deux variantes de réalisation, un prélèvement P est d'abord effectué. Ce prélèvement peut consister par exemple en des feuilles du composé végétal ciblé ou de tout autre prélèvement pour lequel le procédé de l'invention peut s'avérer adapter. Il peut notamment s'agir de cellules d'un être vivant ou de toute autre substance comportant des molécules ADN et susceptible de présenter des espèces biologiques sous une forme active et sous une forme dormante.

E0 : Ce prélèvement est initialement traité en vue d'obtenir un échantillon ECH exploitable et facilement séparable en deux parties A, B de quantités égales.

Ce traitement initial peut consister à broyer le prélèvement puis à réaliser une extraction en vue d'obtenir un liquide prêt à l'analyse. Ce type de traitement est bien connu et n'est pas détaillé dans la présente demande.

L'échantillon ECH obtenu, sous une forme liquide, peut ensuite faire l'objet du procédé de l'invention.

Le procédé de l'invention peut être mis en œuvre en utilisant le même composant micro-fluidique 1 ou en utilisant deux composants micro-fluidiques identiques en parallèle.

Avantageusement, les deux parties A, B de l'échantillon ECH sont injectées dans deux composants identiques en parallèle et les étapes du procédé sont réalisées de manière simultanée dans les deux composants pour analyser les deux parties de l'échantillon. Cette solution permet de fiabiliser les résultats et de minimiser les risques de dégradation de l'échantillon au cours du temps.

Dans les deux variantes décrites ci-dessous, de manière non limitative, on utilise deux composants micro-fluidiques identiques en parallèle, pour analyser de manière distincte une première partie de l'échantillon et une deuxième partie de l'échantillon.

Dans la première variante, en référence à la figure 1, le procédé suit les étapes ci-dessous.

E1 : L'échantillon ECH, contenant les espèces biologiques cibles, est divisé en deux parties A, B de quantités égales.

E2 : La première partie A de l'échantillon traité est injectée en flux dans le premier composant micro-fluidique par son premier canal 14. Les espèces biologiques présentes dans cette première partie d'échantillon restent piégées dans l'espace inférieur 130 de la chambre 13 du composant micro-fluidique, permettant ainsi leur concentration.

Elles sont ensuite avantageusement purifiées par injection d'un liquide tampon par le premier canal du composant, ce liquide traverse la chambre 13 et le filtre 16 et est ensuite évacué via le deuxième canal 15.

E3 : La deuxième partie B de l'échantillon traitée est injectée en flux dans le deuxième composant micro-fluidique par son premier canal 14.

Les espèces biologiques présentes dans cette deuxième partie d'échantillon restent piégées dans l'espace inférieur 130 de la chambre 13 du composant micro-fluidique, permettant ainsi leur concentration.

Elles sont ensuite avantageusement purifiées par injection en flux d'un liquide tampon par le premier canal du composant, ce liquide traverse la chambre 13 et le filtre 16 et est ensuite évacué via le deuxième canal 15.

E4 : La première partie A de l'échantillon ne subit aucun traitement ou au plus un traitement doux T1.

Ce traitement doux T1 peut consister en un simple lavage avec un tampon, par exemple un tampon aqueux avec des surfactants doux (0,01% de tampon Tween 20 - marque déposée). Ce traitement doux T1 ne doit pas permettre de détruire les espèces biologiques cibles.

En cas de lavage, le tampon est injecté en flux à travers le premier canal 14 du composant.

E5 : La deuxième partie B de l'échantillon subit pour sa part un traitement actif T2.

Ce traitement actif T2 permet de casser les molécules d'ADN de la forme active F2 de l'espèce biologique.

Si l'espèce biologique est initialement présente dans sa forme active, les molécules d'ADN F2' cassées lors de l'étape de traitement actif ne sont pas retenues par le filtre 16 du composant lors de l'étape de purification. Elles sont donc évacuées par élution en passant à travers le filtre 16 puis par le deuxième canal 15 du composant micro-fluidique.

Les autres espèces biologiques présentes dans cette deuxième partie B de l'échantillon restent piégées dans l'espace inférieur 130 de la chambre 13 du composant micro-fluidique, permettant ainsi leur concentration.

Les espèces biologiques restantes sont ensuite avantageusement purifiées par injection d'un liquide tampon par le premier canal du composant, ce liquide traverse la chambre et le filtre 16 et est ensuite évacué via le deuxième canal 15.

Ce traitement actif T2 peut être choisi parmi les traitements suivants ou une combinaison d'au moins deux de ces traitements :
- Un traitement chimique,
- Un traitement biochimique,
- Un traitement thermique,
- Un traitement optique,
- Un traitement mécanique.

Le traitement actif T2 est choisi pour être adapté à la destruction de la forme active F2 de l'espèce biologique ciblée dans l'échantillon ECH et au maintien de la forme dormante F1 de l'espèce biologique ciblée. Selon l'espèce biologique et selon sa forme active, on choisit donc un traitement actif le plus efficace possible.

Le traitement actif comporte au moins un traitement chimique ou biochimique de la deuxième partie de l'échantillon, réalisé en flux à travers le composant dans lequel les espèces biologiques F1, F2 ont été piégées lors de l'étape E3 précédente. Chaque traitement actif appliqué peut permettre d'éliminer graduellement la forme active F2 de l'espèce biologique ciblée. La substance est injectée en flux dans le composant 1, à travers son premier canal 14, et vient ainsi directement à la rencontre des espèces biologiques F1, F2 présentes dans l'espace inférieur 130 de la chambre 13 du composant 1.

Dans le cas d'un traitement chimique ou biochimique, il s'agit de choisir une substance qui permettra de casser les molécules d'ADN de la forme active F2 de l'espèce biologique ciblée et de l'ajouter en quantité suffisante à la deuxième partie B de l'échantillon pour obtenir cette destruction, tout en conservant intactes celles de l'espèce biologique dans sa forme dormante F1.

Dans le cas d'un ajout d'un traitement thermique, il s'agira de chauffer la deuxième partie B de l'échantillon à une température suffisante et pendant une durée suffisante pour casser les molécules d'ADN de la forme active F2 de l'espèce biologique cible, tout en conservant intactes celles de l'espèce biologique dans sa forme dormante F1.

Dans le cas d'un ajout d'un traitement optique, il s'agit d'éclairer la deuxième partie B de l'échantillon avec un signal lumineux à une longueur d'onde données, à une intensité donnée et pendant une durée suffisante pour casser les molécules d'ADN de la forme active F2 de l'espèce biologique, tout en conservant intactes celles de l'espèce biologique dans sa forme dormante F1.

Dans le cas d'un ajout d'un traitement mécanique, il s'agira d'appliquer une pression mécanique suffisante sur les espèces biologiques de la deuxième partie B de l'échantillon, par exemple en effectuant une lyse, pour casser les molécules d'ADN de la forme active F2 de l'espèce biologique, tout en conservant intactes celles de la forme dormante F1 de l'espèce biologique.

E6 : Il s'agit d'une étape de détection par amplification biomoléculaire afin de quantifier les espèces biologiques cibles présentes dans la première partie A de l'échantillon biologique. Cette étape est avantageusement mise en œuvre directement dans le composant micro-fluidique.

E7 : Il s'agit d'une étape de détection par amplification biomoléculaire afin de quantifier les espèces biologiques cibles présentes dans la deuxième partie B de l'échantillon biologique. Cette étape est avantageusement mise en œuvre directement dans le composant micro-fluidique.

E8/E9 : Il s'agit de l'étape de traitement des résultats obtenus. Il s'agit en effet de comparer la quantité Q1 d'espèces biologiques obtenue dans la première partie de l'échantillon et la quantité Q2 d'espèces biologiques obtenue dans la deuxième partie de l'échantillon.

Si Q1 et Q2 sont négatifs : on en déduit que l'espèce biologique recherchée est absente de l'échantillon de départ.

Si Q1=Q2 : On en déduit que l'espèce biologique ciblée est bien présente mais qu'elle n'est présente que sous sa forme dormante F1 dans l'échantillon de départ. Si la forme active F2 avait été présente, le traitement actif T2 réalisé sur la deuxième partie de l'échantillon aurait dû permettre de l'éliminer et les deux quantités Q1, Q2 déterminées seraient alors distinctes.

Si Q1>0 et Q2<Q1 : Cela signifie que l'espèce biologique est présente sous sa forme active F2 dans l'échantillon ECH de départ. En effet, le traitement actif T2 a permis de casser les molécules d'ADN de la forme active de l'espèce biologique, celles-ci (référence F2') ont été évacuées du composant micro-fluidique 1 car non retenues par le filtre 16. La quantité Q2 d'espèces biologiques présentes dans la deuxième partie B de l'échantillon est donc plus faible que la quantité Q1 d'espèces biologiques présentes dans la première partie A de l'échantillon.

Dans la deuxième variante de réalisation illustrée par la figure 2, on utilise également deux composants micro-fluidiques identiques en parallèle. De plus, le traitement doux T1 et le traitement actif T2 réalisés respectivement sur la première partie A de l'échantillon et sur la deuxième partie B de l'échantillon peuvent être mis en œuvre préalablement avant l'injection des échantillons dans chacun des deux composants micro-fluidiques employés en parallèle (étapes E20 et E30 sur la figure 2). Le traitement actif T2 peut ensuite être poursuivi en flux pour éliminer les formes actives F2 de l'espèce biologique qui pourraient être encore présentes, après l'injection de la deuxième partie B de l'échantillon dans le composant.

Les étapes E4 et E5 décrites ci-dessus, relatives à l'injection en flux des deux parties A, B de l'échantillon dans chacun des deux composants restent identiques à celles décrites ci-dessus. Le traitement actif T2 ayant été entrepris lors de l'étape E30 sur la deuxième partie B de l'échantillon, les molécules d'ADN F2' ne sont pas retenues par le filtre 16 du composant et sont évacuées du composant 1. Les étapes E40 et E50 consistent à traiter en flux chacun des échantillons piégés lors des étapes E4 et E5 (traitement doux T1 éventuel à l'étape E40 pour la première partie A de l'échantillon et traitement actif T2 en flux à l'étape E50 pour la deuxième partie B de l'échantillon). Le traitement actif T2 permet d'évacuer hors du composant 1 les formes actives résiduelles, au niveau de la deuxième partie B de l'échantillon. Le traitement actif comporte au moins un traitement chimique ou biochimique tel que décrit ci-dessus pour la première variante de réalisation.

Les étapes E6, E7, E8 et E9 décrites ci-dessus pour la première variante de réalisation restent identiques.

### Exemple :

Le principe de l'invention est démontré sur des échantillons de bactéries "Bacillus subtillis (ci-après Bs)" qui existent sous forme de bactéries végétatives (forme active) ou de spores plus résistantes (forme dormante).

Il est connu que les spores et les bactéries végétatives ne sont pas du tout égales face au traitement thermique. Ce traitement est même utilisé pour éliminer toute trace de bactéries dans les spores.

Pour vérifier les principes de l'invention, deux échantillons ont donc été utilisés :
- Un premier échantillon ne comportant que des spores de Bs ;
- Un deuxième échantillon ne comportant que la forme végétative (active) de Bs ;

Pour chacun des deux échantillons, les étapes sont alors les suivantes :
- L'échantillon est divisé en deux parties, première partie A et deuxième partie B, de 0.5mL chacun.
- La première partie A de l'échantillon ne subit aucun traitement ou au plus un traitement doux sans aucun effet sur les espèces biologiques cibles présentes.
- La deuxième partie B de l'échantillon subit un traitement actif sous la forme d'un traitement thermique. Un chauffage de l'échantillon est mené à 80°C pendant 10 minutes.
- Les deux parties de l'échantillon sont injectés dans deux composants micro-fluidiques distincts.
- Dans chacun des deux composants micro-fluidiques, on effectue une lyse des espèces biologiques présentes dans l'espace inférieur de la chambre.
- Dans chacun des composants micro-fluidiques, élution à l'aide d'un mix qPCR (25µL)
- Mise en détection de type PCR des deux éluats pour obtenir les deux quantités d'espèces biologiques cibles, Q1 et Q2 pour chacune des deux parties de l'échantillon.
- Analyse des résultats selon le principe déjà décrit ci-dessus.

Les résultats obtenus sont résumés dans le tableau ci-dessous :

| | | **Partie A** | **Partie B** | Conclusion |
|---|---|---|---|---|
| | | **Sans traitement** | **Traitement actif** | |
| **Echantillon n°1** | Ct qPCR* | 25,2 | Non présente | 100% de formes actives |
| | Quantité | 10080 | 0 | Estimation à 10000 Bs/500µl |
| **Echantillon n°2** | Ct qPCR* | 30,2 | 32,0 | 1/3 de formes dormantes et 2/3 de formes actives |
| | Quantité | 346 | 102 | Estimation à 350 Bs/500µl |

| | | | | |
|---|---|---|---|---|
| *Ct=cycle de seuil qPCR. | | | | |

Dans l'échantillon n°1, on constate donc que les bactéries ont été cassées par le traitement thermique, les molécules d'ADN n'ont pas été retenues dans le composant micro-fluidique ce qui explique que la détection soit négative. On en conclut que l'échantillon n°1 de 10 000 bactéries Bs/500µL est constitué uniquement de l'espèces biologique Bs sous sa forme végétative (active).

Pour l'échantillon n°2, on obtient au total 350bS/500µL environ et on constate après traitement thermique que la quantité présente a fortement baissé. On en conclut donc que 2/3 des espèces biologiques Bs étaient présentes sous la forme végétative (active) dans cette échantillon n°2.

En résumé, l'invention présente ainsi de nombreux avantages parmi lesquels :
- Le principe de détection est simple à mettre en œuvre, ne nécessitant pas de matériel encombrant ;
- Les traitements réalisés en flux permettent d'attaquer toutes les espèces biologiques piégées dans le composant ;
- Il utilise des méthodes classiques de détection ;
- Il est particulièrement fiable et ne nécessite pas l'exécution de modèles mathématiques complexes ;
- Il est facilement adaptable à différents types d'espèces biologiques ;

## Revendications

1. Procédé d'analyse d'un échantillon (ECH) afin de détecter la présence d'une espèce biologique sous une première forme, dite forme active (F2), dans ledit échantillon, ledit échantillon comportant ladite espèce biologique sous une deuxième forme, dite forme dormante (F1) de ladite espèce biologique, distincte de la forme active (F2), **caractérisé en ce que**, à partir d'un même échantillon, il comporte des étapes de :
- Séparation dudit échantillon (ECH) en deux parties (A, B) distinctes de volumes identiques, dites première partie (A) de l'échantillon et deuxième partie (B) de l'échantillon,
- Première concentration des espèces biologiques contenues dans la première partie (A) de l'échantillon,
- Traitement doux (T1) en flux ou aucun traitement de la première partie (A) de l'échantillon,
- Première détection quantitative de ladite espèce biologique dans la première partie (A) de l'échantillon en vue de déterminer une première quantité (Q1) d'espèces biologiques dans la première partie (A) de l'échantillon,
- Deuxième concentration des espèces biologiques contenues dans la deuxième partie (B) de l'échantillon,
- Traitement actif (T2) en flux de la deuxième partie (B) de l'échantillon,
- Deuxième détection quantitative de ladite espèce biologique dans la deuxième partie (B) de l'échantillon en vue de déterminer une deuxième quantité (Q2) d'espèces biologiques dans la deuxième partie de l'échantillon,
- Comparaison entre la première quantité (Q1) obtenue et la deuxième quantité (Q2) obtenue, en vue de déduire la présence ou l'absence de la forme active (F2) de l'espèce biologique dans l'échantillon.

2. Procédé selon la revendication 1, **caractérisé en ce que** le traitement actif de la deuxième partie (B) de l'échantillon comporte au moins un traitement chimique ou biochimique, réalisé par injection en flux d'une substance destructrice de la forme active de l'espèce biologique à la deuxième partie (B) de l'échantillon après concentration, en vue de casser sa membrane externe et libérer ses acides nucléiques.

3. Procédé selon la revendication 2, **caractérisé en ce que** le traitement actif comporte également un ou plusieurs des traitements suivants, ou une combinaison d'au moins deux de ces traitements :
- Traitement thermique,
- Traitement mécanique,
- Traitement optique.

4. Procédé selon la revendication 3, **caractérisé en ce que** le traitement actif est réalisé par lyse des espèces biologiques contre une surface rugueuse.

5. Procédé selon l'une des revendications 3 ou 4, **caractérisé en ce que** le traitement thermique consiste en un chauffage par des moyens de chauffage de la deuxième partie de l'échantillon à une température donnée et pendant une durée déterminée.

6. Procédé selon l'une des revendications 3 à 5, **caractérisé en ce que** le traitement optique consiste à exposer la deuxième partie de l'échantillon à un signal lumineux émis à une longueur d'onde donnée et pendant une durée déterminée.

7. Procédé selon l'une des revendications 1 à 6, **caractérisé en ce que** la première concentration et la deuxième concentration sont mises en œuvre en employant un composant micro-fluidique qui comporte :
- Un boîtier comportant au moins une ouverture,
- Un premier canal (14) ménagé dans ledit boîtier,
- Un deuxième canal (15) ménagé dans ledit boîtier,
- Une chambre (13) dans laquelle débouche le premier canal et le deuxième canal,
- Un filtre (16) séparant ladite chambre en deux espaces distincts de manière à définir un premier espace (130) dans lequel débouche ledit premier canal d'injection et un deuxième espace (131) dans lequel débouche ledit deuxième canal, ledit filtre (16) ayant une porosité adaptée pour retenir les espèces biologiques présentes dans l'échantillon.

8. Procédé selon la revendication 7, **caractérisé en ce que** la première concentration et la deuxième concentration sont mises en œuvre de manière simultanée dans deux composants micro-fluidiques identiques en parallèle.

9. Procédé selon la revendication 7 ou 8, **caractérisé en ce que** le traitement doux (T1) et/ou ledit traitement actif (T2) sont mis en œuvre dans ledit composant micro-fluidique, par injection en flux à travers le premier canal (14) du composant.

10. Procédé selon l'une des revendications 1 à 9, **caractérisé en ce que** la première détection quantitative et la deuxième détection quantitative sont réalisées par amplification biomoléculaire.

11. Procédé selon l'une des revendications 1 à 10, **caractérisé en ce que** l'échantillon est obtenu après une étape de broyage et d'extraction d'un prélèvement à analyser.
